# EUROPEAN PATENT APPLICATION

(11) **EP 2 789 292 A2**
(43) Date of publication of application: **15.10.2014**
(21) Application number: 14163821.3
(22) Date of filing: 08.04.2014
(51) Int. Cl.: A61B 3/117, A61B 3/12, A61B 3/107

(54) **Ophtalmic observation apparatus and method of using the same**

(30) Priority: 10.04.2013 JP 2013081923
(71) Applicant: Hoya Corporation, Shinjuku-ku Tokyo 161-8525 (JP)
(72) Inventor: Shibahara, Yoshitaka, Tokyo, Tokyo 161-8525 (JP); Watanabe, Hiroyuki, Tokyo, Tokyo 161-8525 (JP)
(74) Representative: Schaumburg, Thoenes, Thurn, Landskron, Eckert

(57) **Abstract**

An ophthalmic observation apparatus includes an illumination light supplier which supplies illumination light, of substantially parallel light rays, toward an examinee's eye including an eyeground and an anterior chamber angle, an imager which captures an image of the eye which is illuminated by the illumination light, a rotary driver which rotates the illumination light supplier and the imager about a specified center, a cornea curvature measurer which measures a curvature of a cornea of the examinee's eye, and a controller which sets a curvature center of the cornea as the specified center based on the measured curvature of the cornea. The controller drives the rotary driver to rotate the illumination light supplier and the imager about the specified rotational center and controls the imager to capture an image of the eyeground and the anterior chamber angle illuminated by the illumination light.

## Description

### BACKGROUND OF THE INVENTION

### 1. Field of the Invention

The present invention relates to an ophthalmic observation apparatus for observing the eyeground (ocular fundus) and the anterior chamber angle of the examinee (the eye to be examined) and a method using the same apparatus.

### 2. Description of the Related Art

Ophthalmoscopy (funduscopy, fundoscopy or fundus examination), which is for examining the condition of the eyeground of the examinee (patient), and gonioscopy, which is for examining the condition of the anterior chamber angle of the examinee, are two major supportive examinations for diagnosing eye diseases such as cataract and glaucoma. In the related art, ophthalmoscopy and gonioscopy have been performed using an eyeground observation apparatus (or fundus camera) and an anterior-chamber angle observation apparatus (or goniolens), respectively, which are specially designed and independent of each other, so that the examination system therefor is large and high cost, and requires a long period of time to carry out an examination.

To overcome such problems, an ophthalmic observation apparatus which allows ophthalmoscopy and gonioscopy to be performed in a continuous work-flow manner by focusing illumination light, which is emitted by a lamp or an illuminator toward the retina of the examinee, on the inside of the crystalline lens of the examinee and by rotating the lamp or the illuminator about the center of the focused illumination light in the crystalline lens has been proposed in a brochure of International Publication No. 2012/001381.

However, in the ophthalmic observation apparatus disclosed in the brochure of International Publication No. 2012/001381, the illumination light tends to disperse at the eyeground due to being focused on the inside of the crystalline lens of the examinee; in addition, the operations to concentrate illumination light at a point in the crystalline lens of the examinee and to set the rotational center within the crystalline lens of the examinee are complicated and difficult, so that the precision of ophthalmoscopy and gonioscopy deteriorate.

### SUMMARY OF THE INVENTION

The present invention has been devised while paying attention to the above-described problems and provides an ophthalmic observation apparatus and a method of using the same, each of which makes it possible to reduce the size of the examination system and costs, shorten the time required for the examination, and allow ophthalmoscopy and gonioscopy to be performed with precision in a continuous work-flow manner.

According to an aspect of the present invention, an ophthalmic observation apparatus is provided, including an illumination light supplier which supplies illumination light, consisting of substantially parallel rays of light, toward an eye of an examinee which includes an eyeground and an anterior chamber angle; an imager which captures an image of the eye which is illuminated by the illumination light; a rotary driver which rotates the illumination light supplier and the imager about a specified rotational center; a cornea curvature measurer which measures a curvature of a cornea of the eye of the examinee; and a controller which sets a curvature center of the cornea as the specified rotational center based on the curvature of the cornea measured by the cornea curvature measurer. The controller drives the rotary driver to rotate the illumination light supplier and the imager about the specified rotational center and controls the imager to capture an image of the eyeground and the anterior chamber angle illuminated by the illumination light.

It is desirable for the imager to capture an image of the eyeground when the illumination light, which is supplied by the illumination light supplier, is substantially parallel to a straight line which passes through a surface center of the cornea and the curvature center of the cornea. The imager captures an image of the anterior chamber angle when the illumination light, which is supplied by the illumination light supplier, is inclined to the straight line.

It is desirable for the rotary driver to rotate the illumination light supplier and the imager about the specified rotational center within a range of -70 to +70 degrees relative to the straight line, wherein a rotation angle of the illumination light supplier and the imager about the specified rotational center is 0 degrees when the illumination light is substantially parallel to a straight line which passes through a surface center of the cornea and the curvature center of the cornea.

It is desirable for the ophthalmic observation apparatus to include a rotatable unit which includes the illumination light supplier and the imager, wherein the rotatable unit can be rotatably driven about the specified rotational center by the rotary driver.

In an embodiment, a method of using an ophthalmic observation apparatus, is provided, which includes an illumination light supplier which supplies, toward an eye of an examinee which includes an eyeground and an anterior chamber angle, illumination light consisting of substantially parallel rays of light, an imager which captures an image of the eye which is illuminated by the illumination light, a rotary driver which rotates the illumination light supplier and the imager about a specified rotational center, and a cornea curvature measurer which measures a curvature of a cornea of the eye of the examinee, the method including setting a curvature center of the cornea as the specified rotational center based on the curvature of the cornea measured by the cornea curvature measurer; controlling the imager to capture an image of the eyeground illuminated by the illumination light when the illumination light supplier and the imager are rotated about the specified rotational center by the rotary driver to an eyeground observation position; and controlling the imager to capture an image of the anterior chamber angle illuminated by the illumination light when the illumination light supplier and the imager are rotated about the specified rotational center by the rotary driver to an anterior chamber angle observation position that is different from the eyeground observation position.

According to the present invention, an ophthalmic observation apparatus and a method of using the same, are achieved, which enables the reduction in size of the examination system and costs, shortens the time required for the examination, and allows ophthalmoscopy and gonioscopy to be performed with precision in a continuous work-flow manner.

### BRIEF DESCRIPTION OF THE DRAWINGS

The present invention will be described below in detail with reference to the accompanying drawings in which:

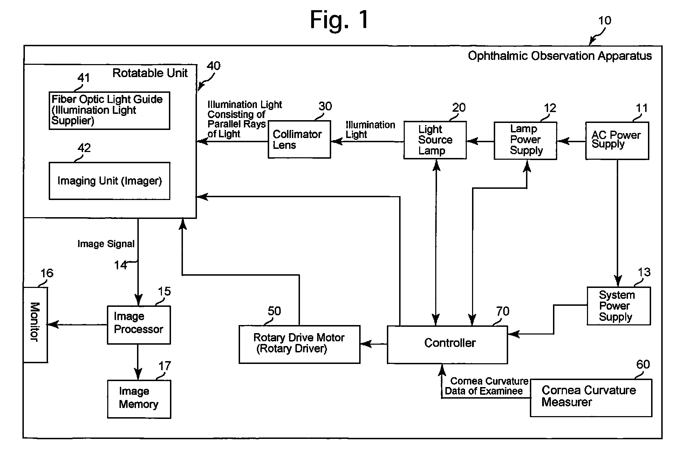

FIG. 1 is a block diagram showing the structure of an embodiment of an ophthalmic observation apparatus according to the present invention;

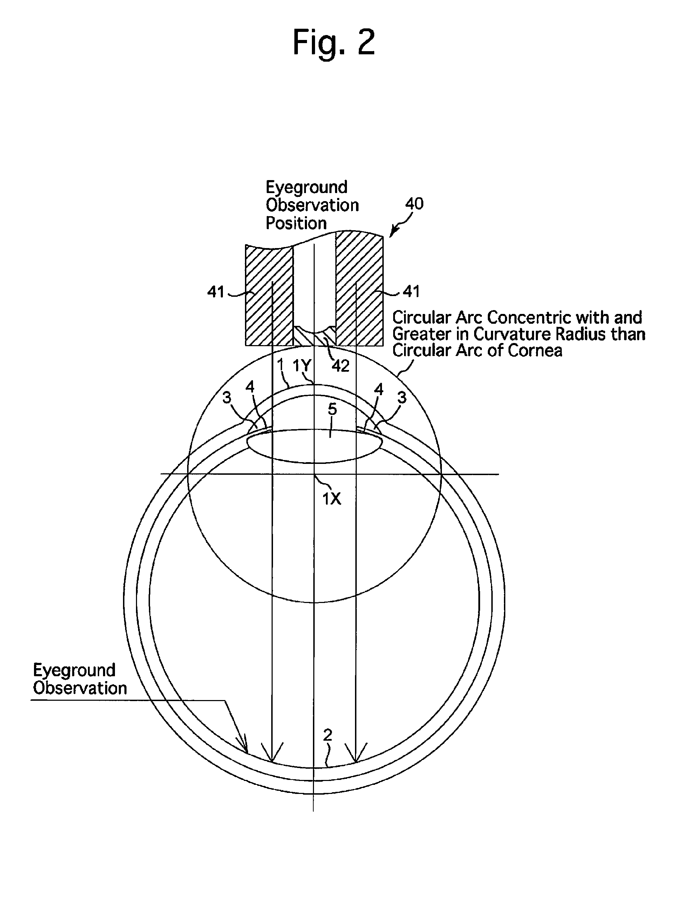

FIG. 2 is a diagram showing a state where the eyeground of the examinee is imaged by an imaging unit in an eyeground observation position where illumination light consisting of substantially parallel rays of light supplied through a fiber optic light guide is substantially parallel to a straight line which passes through the surface center and the curvature center of the cornea of the examinee;

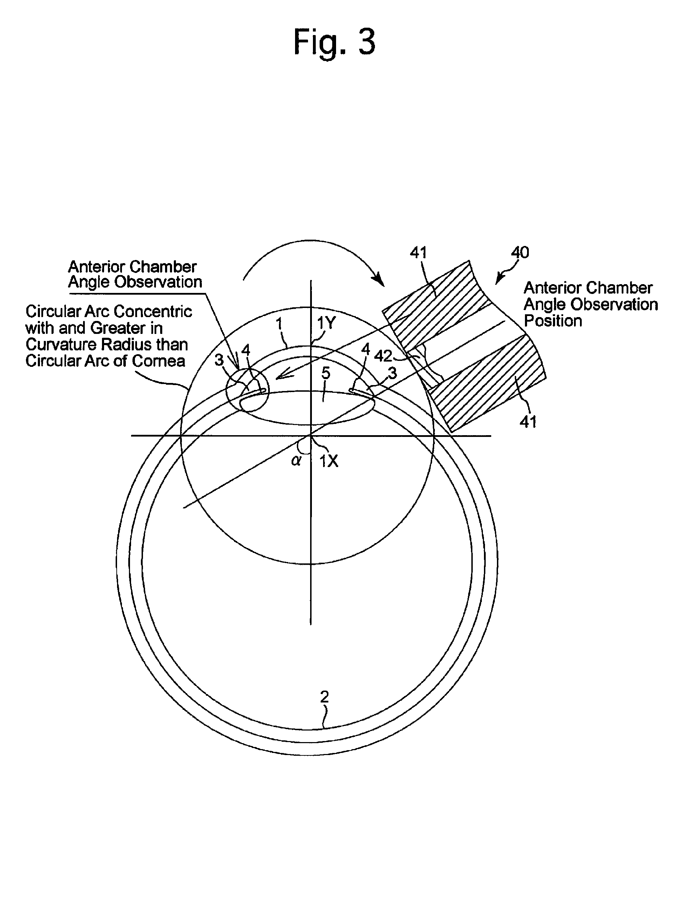

FIG. 3 is a diagram showing a state where the anterior chamber angle of the examinee is imaged by the imaging unit in one of two anterior chamber observation positions where illumination light consisting of substantially parallel rays of light supplied through the fiber optic light guide is inclined to the aforementioned straight line, which passes through the surface center and the curvature center of the cornea of the examinee;

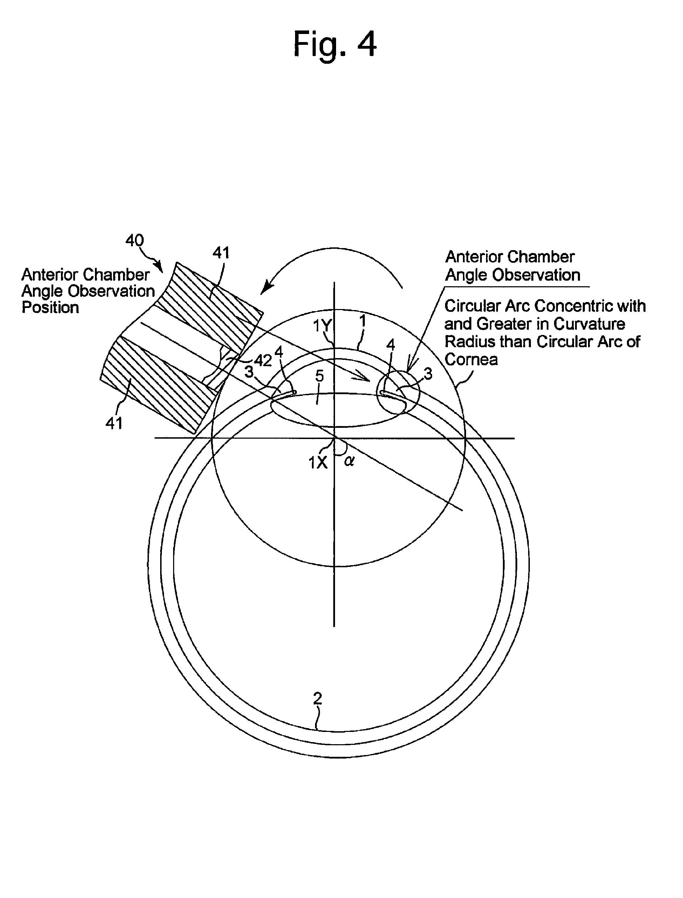

FIG. 4 is a diagram showing a state where the anterior chamber angle of the examinee is imaged by the imaging unit in the other of two anterior-chamber observation positions where illumination light consisting of substantially parallel rays of light supplied through the fiber optic light guide is inclined to the straight line that passes through the surface center and the curvature center of the cornea of the examinee; and

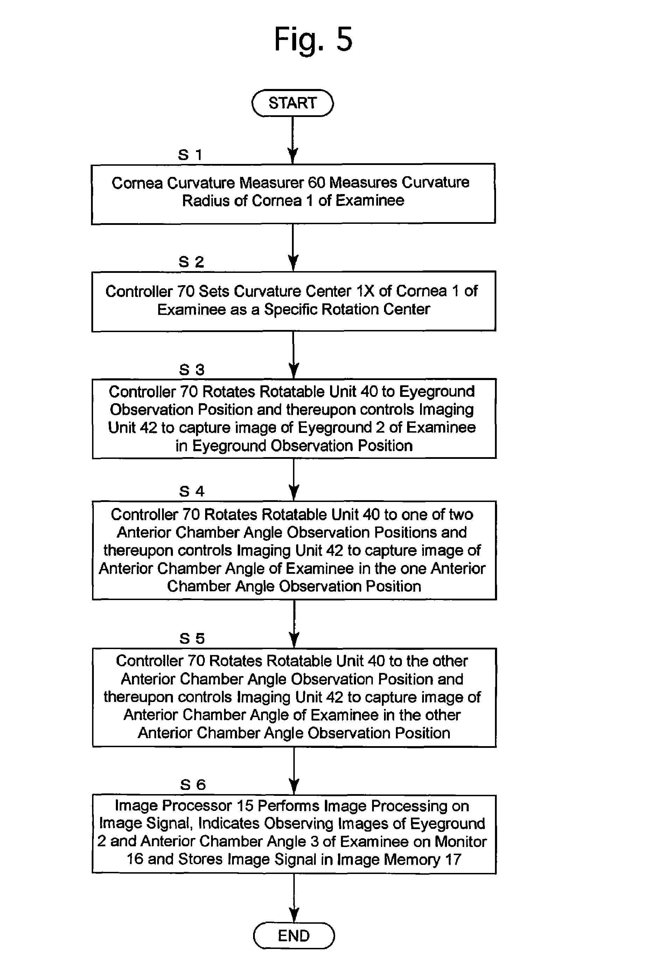

FIG. 5 is a flow chart showing a method of using the ophthalmic observation apparatus according to the present invention.

### DESCRIPTION OF THE PREFERRED EMBODIMENT

An embodiment of an ophthalmic observation apparatus 10 according to the present invention will be hereinafter discussed with reference to FIGS. 1 through 5.

As shown in FIG. 1, the ophthalmic observation apparatus 10 is provided with an AC power supply 11, a lamp power supply 12 and a system power supply 13. The AC power supply 11 serves as a power supply for all the components of the ophthalmic observation apparatus 10, the lamp power supply 12 supplies driving power received from the AC power supply 11 to a lamp system for the ophthalmic observation apparatus 10, and the system power supply 13 supplies driving power received from the AC power supply 11 to a controller (control system) 70 for the ophthalmic observation apparatus 10.

The ophthalmic observation apparatus 10 is provided with a light source lamp 20 which emits illumination light using driving power supplied from the lamp power supply 12 and a collimator lens 30 which collimates the incident light from the light source lamp 20. The light source lamp 20 is made of, e.g., a halogen lamp, a xenon lamp or an LED lamp. Although not shown in the drawings, a rotating diaphragm plate (rotating chopper) for adjusting the light quantity of illumination light which is emitted by the light source lamp 20 is installed between the light source lamp 20 and the collimator lens 30.

As shown in FIGS. 1 through 4, the ophthalmic observation apparatus 10 is provided with a rotatable unit 40 on which the illumination light emitted from the light source lamp 20 and collimated by the collimator lens 30 is incident. The rotatable unit 40 is provided with a cylindrical (ring-shaped) fiber optic light guide (illumination light supplier) 41 and an imaging unit (imager) 42 which is installed in the core of the fiber optic light guide 41. The fiber optic light guide 41 supplies illumination light consisting of substantially parallel rays of light and having a ring shape in cross section toward the eye of the examinee. The imaging unit 42 is a combination of an objective optical system and a solid-state image pickup device and images the examinee's eye (ocular fundus, anterior chamber angle, etc.) which is illuminated by illumination light (consisting of substantially parallel rays of light) supplied through the fiber optic light guide 41. Namely, the illumination light (consisting of substantially parallel rays of light) which emerges from the front end (the end closest to the examinee) of the fiber optic light guide 41 illuminates the examinee's eye (ocular fundus, anterior chamber angle, etc.), and the reflected light is imaged and converted into an image signal by the imaging unit 42. This image signal is transmitted to an image processor 15 provided as a component of the ophthalmic observation apparatus 10 via an image signal transmission cable 14, and a predetermined image processing is performed on the image signal thus transmitted in the image processor 15. Thereupon, this image signal is visually indicated as an observing image on a monitor 16 which is provided as a component of the ophthalmic observation apparatus 10, and the same image signal is stored in an image memory 17 connected to the image processor 15.

As shown in FIG. 1, the ophthalmic observation apparatus 10 is provided with a rotary drive motor (rotary driver) 50 which rotates the rotatable unit 40 (the fiber optic light guide 41 and the imaging unit 42) about a specific point. Although the rotary drive motor 50 is illustrated as an external component of the rotatable unit 40 in the block diagram of FIG. 1 of the present embodiment of the ophthalmic observation apparatus, a different embodiment of the ophthalmic observation apparatus in which the rotary drive motor 50 is provided as an internal component of the rotatable unit 40 is also possible.

As shown in FIG. 1, the ophthalmic observation apparatus 10 is provided with a cornea curvature measurer 60 for measuring the curvature of a cornea 1 (see FIGS. 2 through 4) of the examinee. The cornea curvature measurer 60 can be configured of, e.g., an autokeratometer which projects light for measurement onto the center of the cornea 1, captures the reflected light by the light receiving surface of a CCD camera and automatically computes the curvature of the cornea 1; however, the configuration of the cornea curvature measurer 60 is flexible so long as the cornea curvature measurer 60 can measure the curvature of the cornea 1 of the examinee.

As shown in FIG. 1, the ophthalmic observation apparatus 10 is provided with the controller 70, which controls operations of the overall components of the ophthalmic observation apparatus 10. For instance, the controller 70 is connected to the lamp power supply 12 and the light source lamp 20 to control the ON/OFF operation of the light source lamp 20 and the light quantity (luminous intensity) of illumination light emitted by the light source lamp 20.

The controller 70 captures observing images of an eyeground 2 and an anterior chamber angle 3 of the examinee by controlling operations of the rotatable unit 40 (the fiber optic light guide 41 and the imaging unit 42) and the rotary drive motor 50 based on the curvature of the cornea 1 of the examinee which has been measured by the cornea curvature measurer 60 (see FIGS. 2 through 4). The term "the eyeground 2 of the examinee" denotes the blood vessels, the retina and the optic nerves in the ocular fundus of the examinee, and the term "the anterior chamber angle 3 of the examinee" denotes a point of intersection of the cornea 1 with an iris 4 of the eye. The reference numeral 5 in FIGS. 2 through 4 designates the crystalline lens of the eye of the examinee.

As shown in FIGS. 2 through 4, the controller 70 sets a curvature center 1X of the cornea 1 of the examinee as a specified rotational center based on the curvature of the cornea 1 of the examinee measured by the cornea curvature measurer 60, drives the rotary drive motor 50 to rotate the rotatable unit 40 (the fiber optic light guide 41 and the imaging unit 42) about the specified rotational center 1X, and controls the imaging unit 42 to capture an image of the eyeground 2 and the anterior chamber angle 3 illuminated by illumination light consisting of substantially parallel rays of light which is supplied through the fiber optic light guide 41.

More specifically, assuming that the angle of rotation of the rotatable unit 40 is 0 degrees when illumination light consisting of substantially parallel rays of light, which is supplied through the fiber optic light guide 41, is substantially parallel to a straight line which passes through a surface center 1Y of the cornea 1 of the examinee and the curvature center (specified rotational center) 1X of the cornea 1 of the examinee (as shown in FIG. 2), the controller 70 can rotate the rotatable unit 40 (the fiber optic light guide 41 and the imaging unit 42) by driving the rotary drive motor 50 within the range of ±α° relative to the straight line (i.e., the range of 2α° in total) (see FIGS. 3 and 4). In the following descriptions, the position of the rotatable unit 40 (the fiber optic light guide 41 and the imaging unit 42) in which illumination light consisting of substantially parallel rays of light, which is supplied through the fiber optic light guide 41, is substantially parallel to a straight line which passes through the surface center 1Y and the curvature center 1X of the cornea 1 will be referred to as "eyeground observation position" (the rotatable unit 40 is in this position in FIG. 2), and the position of the rotatable unit 40 (the fiber optic light guide 41 and the imaging unit 42) in which the rotatable unit 40 (the fiber optic light guide 41 and the imaging unit 42) has been rotated at an angle of α° from the eyeground observation position toward either side thereof (clockwise or counterclockwise with respect to FIG. 2) will be referred to as "anterior chamber angle observation position" (the rotatable unit 40 is in this position in each of FIGS. 3 and 4). Namely, the rotatable unit 40 can selectively be positioned at the two anterior chamber angle observation positions shown in FIGS. 3 and 4, respectively. The controller 70 controls the imaging unit 42 to capture an image of the eyeground 2 when the rotatable unit 40 (the fiber optic light guide 41 and the imaging unit 42) is in the eyeground observation position (see FIG. 2), whereas the controller 70 controls the imaging unit 42 to capture an image of the anterior chamber angle 3 from two different angles when the rotatable unit 40 (the fiber optic light guide 41 and the imaging unit 42) is in the two anterior chamber angle observation positions, respectively (see FIGS. 3 and 4). The angle α° of the rotatable unit 40 (the fiber optic light guide 41 and the imaging unit 42) can be set within a range of, e.g., -70 to +70 degrees in accordance with various conditions such as the distance from the rotatable unit 40 (the fiber optic light guide 41 and the imaging unit 42) to the examinee's eye.

A method of using the ophthalmic observation apparatus 10 that has the above described structure will be hereinafter discussed with reference to the flow chart shown in FIG. 5.

First, at step S1, the cornea curvature measurer 60 measures the curvature of the cornea 1 of the examinee and sends data on this measured curvature, as cornea curvature data, to the controller 70.

Subsequently, at step S2, based on the curvature of the cornea 1 of the examinee measured by the cornea curvature measurer 60, the controller 70 sets the curvature center 1X of the cornea 1 of the examinee as a specified rotational center for the rotatable unit 40 (the fiber optic light guide 41 and the imaging unit 42).

Subsequently, at step S3, the controller 70 drives the rotary drive motor 50 to rotate the rotatable unit 40 (the fiber optic light guide 41 and the imaging unit 42) about the specified rotational center (the curvature center 1X) to the eyeground observation position and thereupon controls the imaging unit 42 to capture an image of the eyeground 2 of the examinee in the eyeground observation position (see FIG. 2).

Subsequently, at steps S4 and S5, the controller 70 drives the rotary drive motor 50 to rotate the rotatable unit 40 (the fiber optic light guide 41 and the imaging unit 42) about the specified rotational center (the curvature center 1X), firstly, to one of the two anterior chamber angle observation positions to image the anterior chamber angle 3 in this one angle observation position, and secondly, to the other anterior chamber angle observation position to image the anterior chamber angle 3 in the other angle observation position (see FIGS. 3 and 4).

The above described operations at steps S3, S4 and S5 do not necessarily have to be performed in that specific order; namely, the order of these operation to be performed can be changed as appropriate.

Lastly, at step S6, the image processor 15 performs a predetermined image processing operation on the image signal representing images taken (captured) by the imaging unit 42, thereafter indicates the observing images of the eyeground 2 and the anterior chamber angle 3 of the examinee on the monitor 16, and stores the image signal in the image memory 17.

The presence or absence of eye diseases such as cataract and glaucoma and conditions of such eye diseases can be made known by a medical doctor(s) observing and examining the observing images of the eyeground 2 and the anterior chamber angle 3 of the examinee on the monitor 16. For instance, in the case of cataract, characteristics of retinitis pigmentosa (pigmentary degeneration of the retina) can be seen in the observing image of the eyeground, and haze appears in the observing image of the anterior chamber angle 3. The eyeground 2 contains cone cells (corns) and rod cells (rods), which are sensitive to light, in the retina. Cone cells are responsible for color vision as well as sharpness of vision (eyesight), whereas rod cells are used in peripheral vision and function in less intense light than cone cells, thus being responsible for night vision. In general, it is said that an abnormality appears in cone cells after a disorder of rod cells occurs and that a cataract is caused by a large abnormality in rod cells.

As described above, according to the present embodiment of the ophthalmic observation apparatus 10, the controller 70 sets the curvature center 1X of the cornea 1 of the examinee as a specified rotational center based on the curvature of the cornea 1 of the examinee measured by the cornea curvature measurer 60, drives the rotary drive motor 50 to rotate the rotatable unit 40 (the fiber optic light guide 41 and the imaging unit 42) about the specified rotational center 1X, and controls the imaging unit 42 to capture an image of the eyeground 2 and the anterior chamber angle 3 illuminated by illumination light, consisting of substantially parallel rays of light, which is supplied through the fiber optic light guide 41. This makes it possible to reduce the size of the examination system and costs, shorten the time required for the examination, and allow ophthalmoscopy and gonioscopy to be performed with precision in a continuous work-flow manner.

More specifically, the eyeground observation position and the two anterior chamber angle observation positions are defined by rotating the rotatable unit 40 (the fiber optic light guide 41 and the imaging unit 42) so as to swing along a circular arc which is concentric with and greater in radius of curvature than the circular arc of the cornea 1, and images of the eyeground 2 and the anterior chamber angle 3 of the examinee are captured, accordingly, the examination system is small in size and low in cost. Moreover, the time required for the examination is short, and ophthalmoscopy and gonioscopy can be performed with precision in a continuous work-flow manner. In addition, since illumination light supplied toward the examinee's eye is collimated into substantially parallel rays of light to prevent the illumination light from dispersing at the retina of the examinee, and also since the controller 70 sets the curvature center 1X of the cornea 1 of the examinee as a specified rotational center for the rotatable unit 40 (the fiber optic light guide 41 and the imaging unit 42) based on the curvature of the cornea 1 of the examinee measured by the cornea curvature measurer 60, ophthalmoscopy and gonioscopy can be performed with higher precision. Additionally, since the curvature center 1X of the retina 1 varies depending on the examinee, safety of the examination using the ophthalmic observation apparatus 10 can be secured simply by setting the curvature center 1X of the cornea 1 of each examinee as a specified rotational center for the rotatable unit 40 (the fiber optic light guide 41 and the imaging unit 42). In practice, since the illumination light supplied toward the examinee's eye is dispersed in the leftward/rightward direction with respect to FIGS. 2 through 4 due to lens effect of the crystalline lens 5, the eyeground 2 of the examinee can be widely observed.

Obvious changes may be made in the specific embodiment of the present invention described herein, such modifications being within the spirit and scope of the invention claimed. It is indicated that all matter contained herein is illustrative and does not limit the scope of the present invention.

## Claims

1. An ophthalmic observation apparatus comprising:
an illumination light supplier which supplies illumination light, consisting of substantially parallel rays of light, toward an eye of an examinee which includes an eyeground and an anterior chamber angle;
an imager which captures an image of said eye which is illuminated by said illumination light;
a rotary driver which rotates said illumination light supplier and said imager about a specified rotational center;
a cornea curvature measurer which measures a curvature of a cornea of said eye of said examinee; and
a controller which sets a curvature center of said cornea as said specified rotational center based on said curvature of said cornea measured by said cornea curvature measurer,
wherein said controller drives said rotary driver to rotate said illumination light supplier and said imager about said specified rotational center and controls said imager to capture an image of said eyeground and said anterior chamber angle illuminated by said illumination light.

2. The ophthalmic observation apparatus according to claim 1, wherein said imager captures an image of said eyeground when said illumination light, which is supplied by said illumination light supplier, is substantially parallel to a straight line which passes through a surface center of said cornea and said curvature center of said cornea, and
wherein said imager captures an image of said anterior chamber angle when said illumination light, which is supplied by said illumination light supplier, is inclined to said straight line.

3. The ophthalmic observation apparatus according to claim 1, wherein said rotary driver rotates said illumination light supplier and said imager about said specified rotational center within a range of -70 to +70 degrees relative to said straight line, wherein a rotation angle of said illumination light supplier and said imager about said specified rotational center is 0 degrees when said illumination light is substantially parallel to a straight line which passes through a surface center of said cornea and said curvature center of said cornea.

4. The ophthalmic observation apparatus according to claim 1, further comprising a rotatable unit which includes said illumination light supplier and said imager,
wherein said rotatable unit can be rotatably driven about said specified rotational center by said rotary driver.

5. A method of using an ophthalmic observation apparatus, which includes an illumination light supplier which supplies, toward an eye of an examinee which includes an eyeground and an anterior chamber angle, illumination light consisting of substantially parallel rays of light, an imager which captures an image of said eye which is illuminated by said illumination light, a rotary driver which rotates said illumination light supplier and said imager about a specified rotational center, and a cornea curvature measurer which measures a curvature of a cornea of said eye of said examinee, said method comprising:
setting a curvature center of said cornea as said specified rotational center based on said curvature of said cornea measured by said cornea curvature measurer;
controlling said imager to capture an image of said eyeground illuminated by said illumination light when said illumination light supplier and said imager are rotated about said specified rotational center by said rotary driver to an eyeground observation position; and
controlling said imager to capture an image of said anterior chamber angle illuminated by said illumination light when said illumination light supplier and said imager are rotated about said specified rotational center by said rotary driver to an anterior chamber angle observation position that is different from said eyeground observation position.
